# FASCICULE DE BREVET EUROPEEN

(11) **EP 0 885 234 B1**
(45) Date de publication et mention de la délivrance du brevet: **15.05.2002**
(21) Numéro de dépôt: 97907141.2
(22) Date de dépôt: 27.02.1997
(51) Int. Cl.: C07H 17/08, A61K 31/70

(54) **NOUVEAUX DERIVES DE L'ERYTHROMYCINE, LEUR PROCEDE DE PREPARATION ET LEUR APPLICATION COMME MEDICAMENTS**
ERYTHRAMICIN-DERIVATE, IHRE VERFAHREN ZUR HERSTELLUNG UND IHRE VERWENDUNG ALS ARZNEIMITTEL
NOVEL ERYTHROMYCIN DERIVATIVES, METHOD FOR PREPARING SAME, AND USE THEREOF AS DRUGS

(30) Priorité: 28.02.1996 FR 9602472
(43) Date de publication de la demande: 23.12.1998
(73) Titulaire: Aventis Pharma S.A., 92160 Antony (FR)
(72) Inventeur: AGOURIDAS, Constantin, F-94130 Nogent-sur-Marne (FR); CHANTOT, Jean-François, F-94130 Nogent-sur-Marne (FR)
(86) Numéro de dépôt international: FR9700351
(87) Numéro de publication internationale: WO9731929

(56) Documents cités:
- EP-A- 0 487 411
- EP-A- 0 716 093
- US-A- 5 444 051
- R.A.LeMAHIEU et al. J.Med.Chem. 17/9, 953 (1974)

## Description

La présente invention concerne de nouveaux dérivés de l'érythromycine, leur procédé de préparation et leur application comme médicaments.

L'invention a pour objet les composés choisis dans le groupe constitué par les composés de formule (I) : 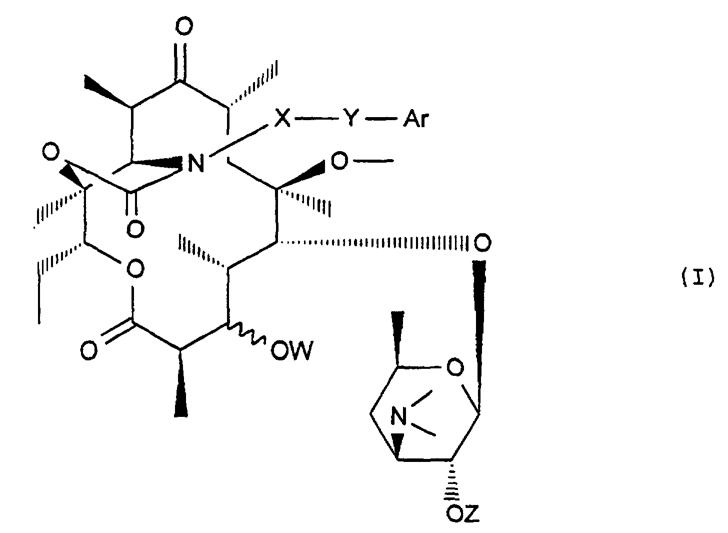 dans lesquels :
X représente un radical CH₂ ou SO₂ ou un atome d'oxygène,
Y représente un radical (CH₂)ₘ-(CH=CH)ₙ(CH₂)ₒ, avec m + n + o ≤ 8, n = 0 ou 1,
Ar représente un radical hétéroaryle éventuellement substitué,
W représente un atome d'hydrogène, ou le reste d'une fonction carbamate 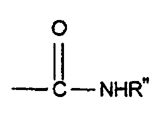 dans lequel R" représente un radical alkyle renfermant jusqu'à 8 atomes de carbone ou un radical aryle, éventuellement substitué,
Z représente un atome d'hydrogène ou le reste d'un acide ainsi que leurs sels d'addition avec les acides, et la 11,12-didéoxy-3-de((2,6-didéoxy-3-C-méthyl-3-O-méthyl-alpha-L-ribohexopyranosyl)oxy)-3-hydroxy-6-O-méthyl-12,11-(oxycarbonyl(2-(3-(4-quinoléinyl)propyl) hydrazono)) érythromycine.

Parmi les sels d'addition avec les acides, on peut citer les sels formés avec les acides acétique, propionique, trifluoroacétique, maléique, tartrique, méthanesulfonique, benzènesulfonique, p-toluènesulfonique, et spécialement les acides stéarique, éthylsuccinique ou laurylsulfonique.

Le radical hétéroaryle peut être un radical hétérocyclique substitué ou non comme le radical thiényle, furyle, pyrolyle, thiazolyle, oxazolyle, imidazolyle, thiadiazolyle, pyrazolyle ou isopyrazolyle, un radical pyridyle, pyrimidyle, pyridazinyle ou pyrazinyle, ou encore un radical indolyle benzofurannyle, benzothiazyle ou quinoléinyle.

Ces radicaux hétéroaryles peuvent comporter un ou plusieurs groupements choisis dans le groupe constitué par les radicaux hydroxyle, les atomes d'halogène, les radicaux NO₂, les radicaux C≡N, les radicaux alkyle, alkényle ou alkynyle, O-alkyle, O-alkényle ou O-alkynyle, S-alkyle, S-alkényle ou S-alkynyle et N-alkyle, N-alkényle ou N-alkynyle, renfermant jusqu'à 12 atomes de carbone éventuellement substitués par un ou plusieurs atomes d'halogène, le radical 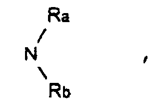 Rₐ et R_{b} identiques ou différents, représentant un atome d'hydrogène ou un radical alkyle renfermant jusqu'à 12 atomes de carbone, le radical 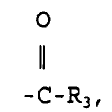 R₃ représentant un radical alkyle renfermant jusqu'à 12 atomes de carbone, ou un radical aryle ou hétéroaryle à 5 ou 6 chaînons comportant un ou plusieurs hétéro-atomes, éventuellement substitués par un ou plusieurs des substituants mentionnés ci-dessus.

Comme hétérocycle préféré, on peut citer entre autres : 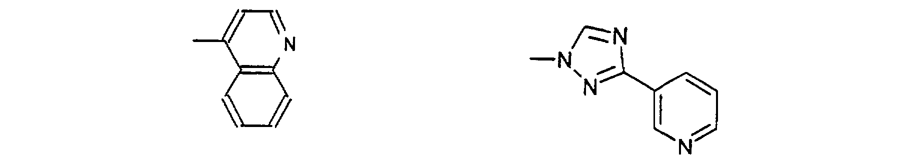 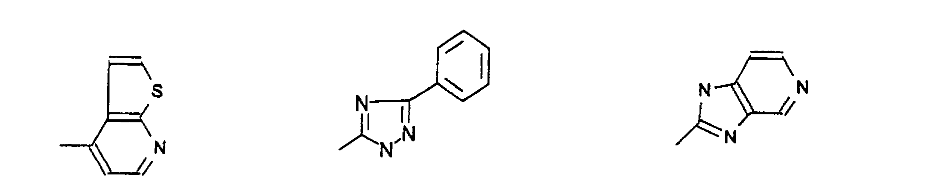 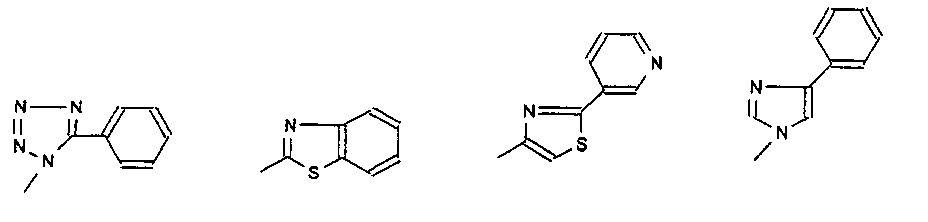 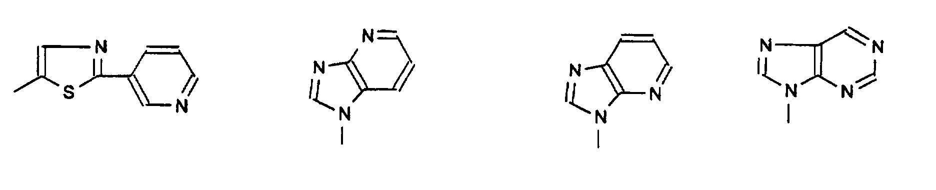 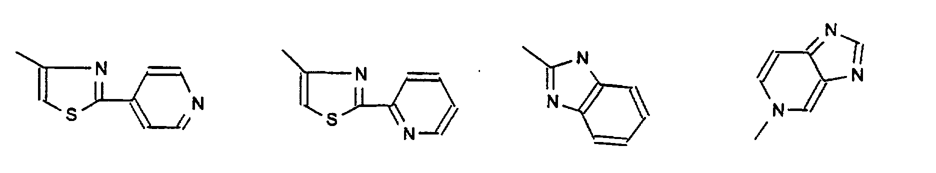 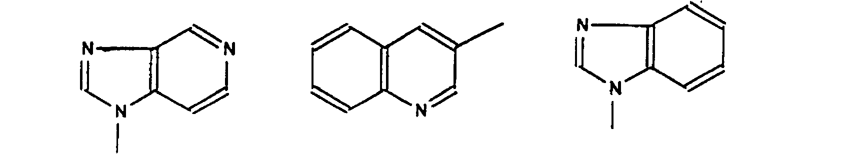 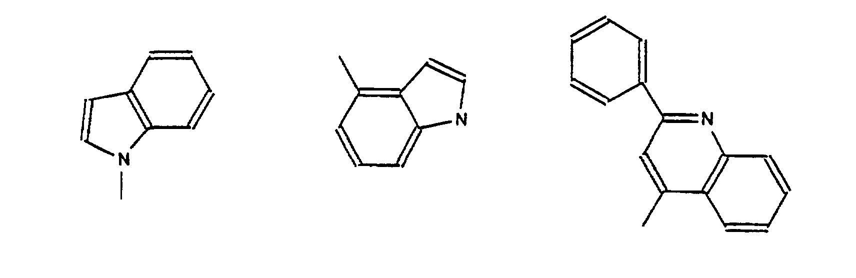 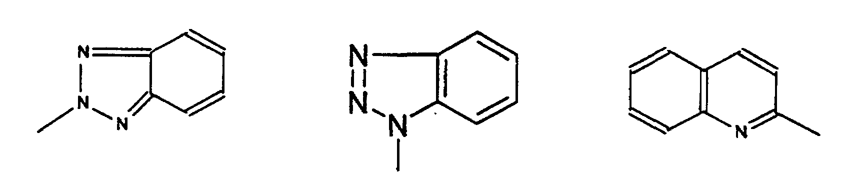 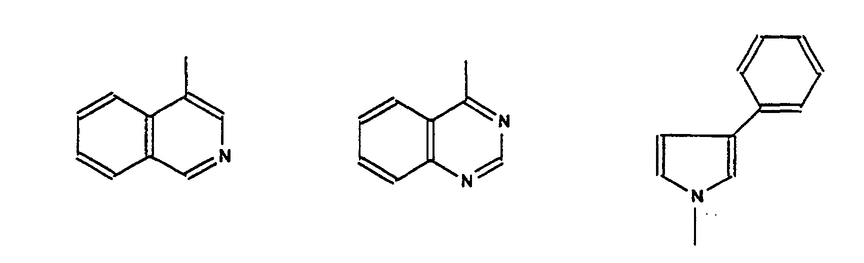 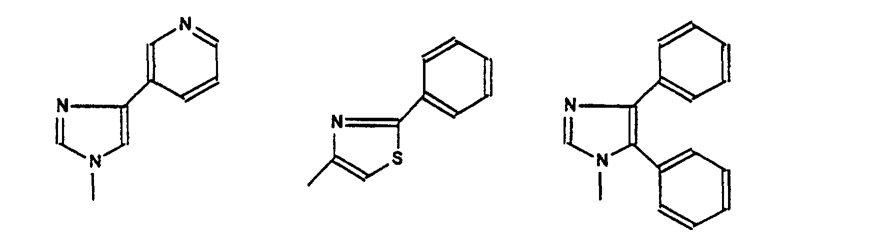 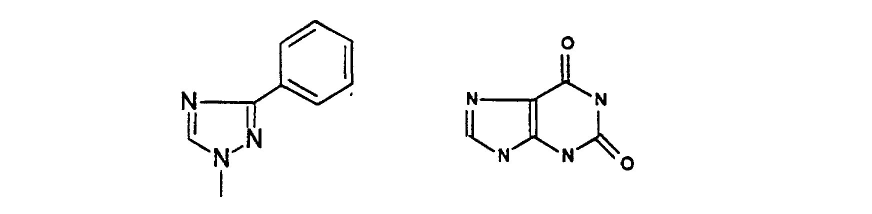 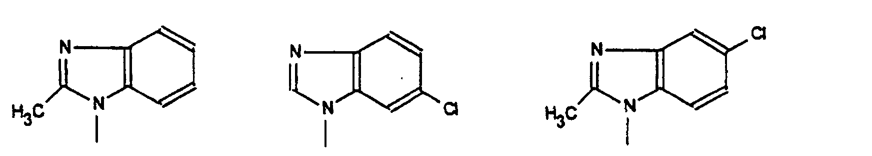 et les radicaux hétérocycliques envisagés dans les demandes de brevets européens 487411, 596802, 676409 et 680967. Ces radicaux hétérocycliques préférés pouvent être substitués par un ou plusieurs groupements fonctionnels.

L'invention a plus particulièrement pour objet les composés de formule (I) dans lesquels Z représente un atome d'hydrogène, ceux dans lesquels W représente un atome d'hydrogène, ceux dans lesquels X représente un radical CH₂, ceux dans lesquels Y représente un radical (CH₂)₃, (CH₂)₄ ou (CH₂)₅.

Parmi les composés préférés de l'invention, on peut citer les composés de formule (I), dans lesquels Ar représente un radical 4-quinoléinyle éventuellement mono ou poly-substitué sur l'un et/ou l'autre des 2 cycles de la quinoléine, et tout particulièrement les composés de formule (I) dans lesquels Ar représente un radical 4-quinoléinyle non substitué, ou encore par exemple les composés de formule (I), dans lesquels Ar représente un radical : 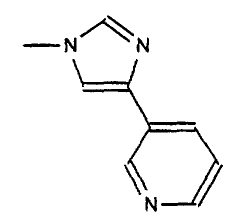 éventuellement substitué.

Parmi les composés préférés de l'invention, on peut citer naturellement les produits dont la préparation est donnée ci-après dans la partie expérimentale.

Les produits de formule générale (I) possèdent une très bonne activité antibiotique sur les bactéries gram ^{⊕} telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumonies, suppuration pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma, ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits des exemples 1 ou 2 et leurs sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 500 mg par jour par voie orale, chez l'adulte pour le produit de l'exemple 1 ou de l'exemple 2.

L'invention a également pour objet un procédé de préparation, caractérisé en ce que l'on soumet un composé de formule (II) : 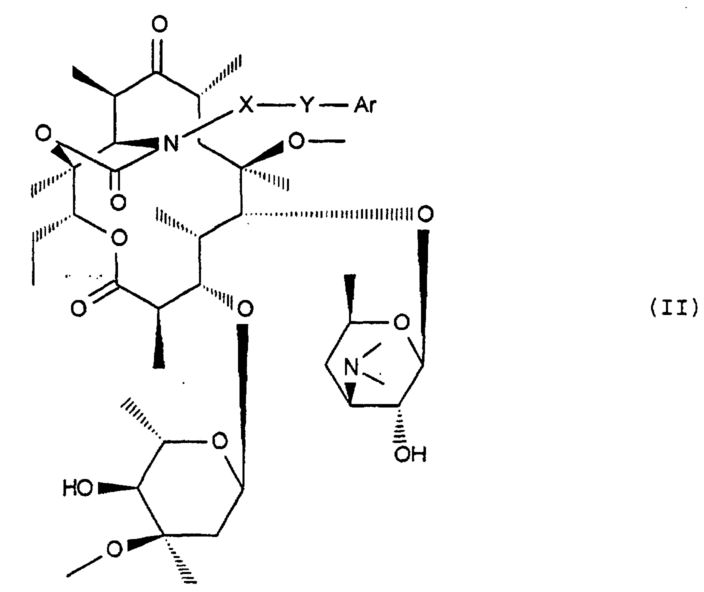 dans lequel X, Y et Ar sont définis comme ci-dessus, à l'action d'un agent de clivage du cladinose pour obtenir le composé de formule (I_{A}) correspondant : 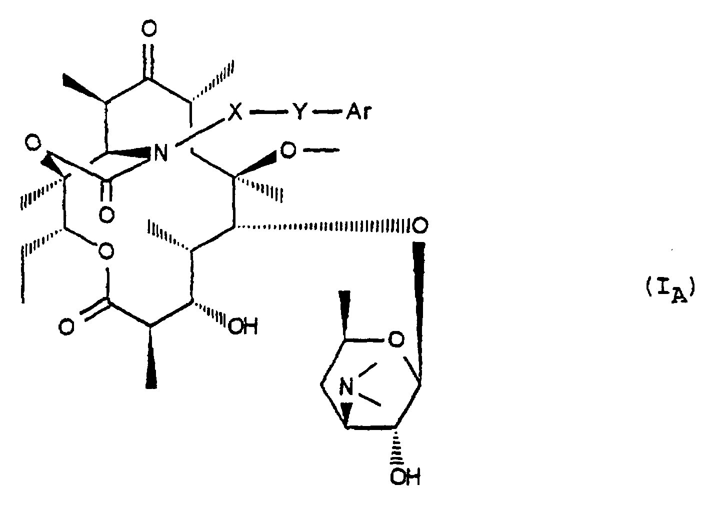 dans lequel X, Y et Ar conservent leur signification précédente que l'on soumet si désiré à l'action d'un agent d'estérification ou à l'action d'un agent susceptible d'introduire le radical carbamate.

Dans un mode de réalisation préféré le clivage du cladinose en 3 est réalisé au moyen d'un acide.

Les produits de formule (II) utilisés comme produits de départ, sont des produits qui peuvent être préparés selon le procédé décrit dans la demande de brevet européen 076093 déposée le 6 décembre 1995 par la Société ROUSSEL UCLAF.

Les produits de formule (II) peuvent être préparés selon un procédé caractérisé en ce que l'on soumet un composé de formule (III) : 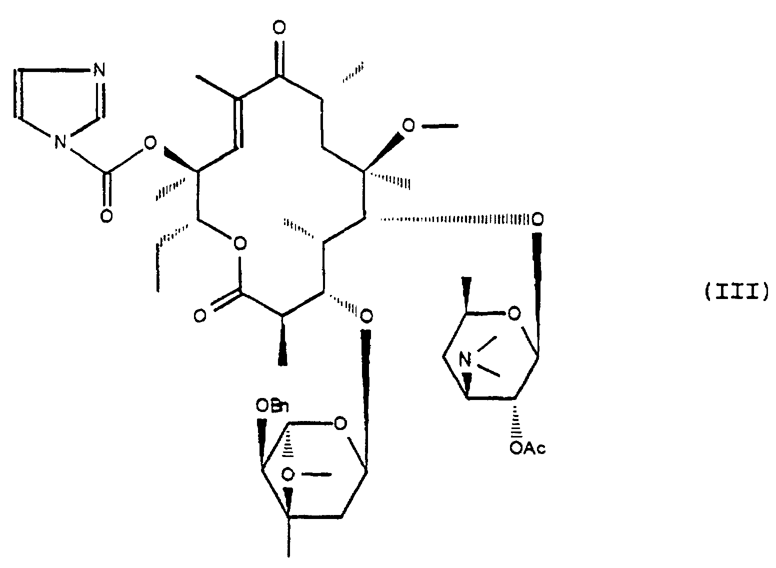 dans laquelle Bn représente un radical benzyloxycarbonyle et Ac un radical acyle renfermant de 2 à 20 atomes de carbone, à l'action d'un composé de formule (IV) :

R₃NH₂ (IV)

dans laquelle R₃ représente le radical X-Y-Ar,
X, Y et Ar étant définis comme précédemment pour obtenir le composé de formule (V) : 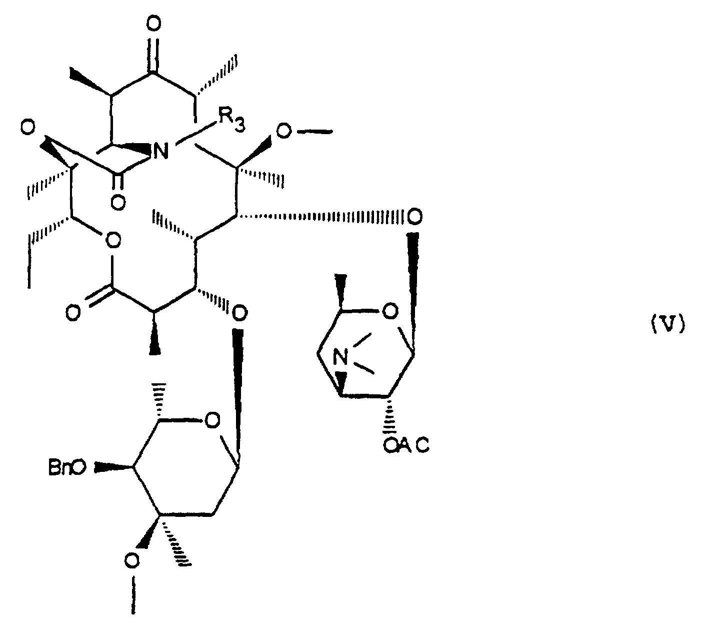 que l'on soumet si désiré à l'action d'un agent de clivage de la fonction ester en 2' pour obtenir le composé 2'-OH correspondant, puis soumet si désiré le composé ainsi obtenu à l'action d'un agent de réduction pour effectuer le clivage du groupement benzyloxy carbonyle en 4" et obtenir le produit de formule (II).

Les composés de formule (III) utilisés comme produits de départ sont des produits connus décrits dans le brevet européen 0 248 279.

Les amines de formule (IV) sont connus d'une façon générale et peuvent être préparés selon les procédés décrits dans J. Med. Chem. (1982) vol. 25, p. 947 et suivantes ou encore Tetrahedron Letters vol. 32 n° 14, p. 1699, 1702 (1991).

Le clivage de l'acétate en 2' est réalisé à l'aide du méthanol.

Le clivage du groupe benzyloxycarbonyle en 4" est réalisé par réduction, par exemple au moyen de l'hydrogène en présence d'un catalyseur au palladium.

La salification est réalisée au moyen d'acide selon les procédés classiques.
Le brevet européen n° EP 0 487 411 décrit des dérivés de l'érythromycine apparentés aux composés de formule I ci-dessus, et notamment, dans son exemple 10, des composés comportant en position 11 - 12 un pont oxycarbonylimino portant sur l'atome d'azote un radical aralkyle et plus particulièrement 1 phénylbutyl et, en position 3, soit un radical alpha-hydroxy, soit un radical céto. Le composé 3-OH est un intermédiaire alors que le 3-céto est un composé final thérapeutiquement actif.

Les exemples suivants illustrent l'invention sans toutefois la limiter.

### EXEMPLE 1 : 11,12-didéoxy-3-de((2,6-didéoxy-3-C-méthyl-3-O-méthyl-alpha-L-ribohexopyranosyl)oxy)-3-hydroxy-6-O-méthyl-12,11-(oxycarbonyl(2-(3-(4-quinoléinyl)propyl)hydrazono))-érythromycine

On ajoute 1 cm³ d'une solution d'acide chlorhydrique dans l'éthanol (22,6 g/l) dans une solution renfermant 250 mg de 11,12-didéoxy-6-O-méthyl-12,11-(oxycarbonyl(2-(3-(4-quinoléinyl) propyl) hydrazono) érythromycine. On agite le mélange réactionnel à la température ambiante pendant une heure. On versedans l'eau, extrait à l'acétate d'éthyle, reprend la phase aqueuse, amène en milieu basique, extrait à l'acétate d'éthyle, sèche, filtre, évapore à sec. On obtient ainsi 271 mg d'un produit que l'on dissout dans l'acétate d'éthyle, on ajoute ensuite de l'éther sulfurique jusqu'à ce que le produit cristallise. On lave à l'éther sulfurique, obtient 169 mg que l'on recristallise. On obtient après séchage à 80°C, 120 mg de produit fondant à 166°C.

Le produit 11,12-didéoxy-6-O-méthyl-12,11-(oxycarbonyl(2-(3-(4-quinoléinyl) propyl) hydrazono) érythromycine a été préparé comme suit :

### STADE A : Préparation de 11,12-didéoxy 6-O-méthyl 12,11-(oxycarbonyl (2-hydrazono)) érythromycine (produit P)

### a) Condensation

On plonge dans un bain à 80°C durant 10 mn, un mélange de 3 g de 2'-acétate 12-(1H-imidazole-1-carboxylate 4"-(phénylméthylcarbonate) de 10,11-didéhydro 11-déoxy 6-O-méthyl érythromycine, 3 ml d'hydrate d'hydrazine, 30 ml d'acétonitrile et 492 mg de carbonate de césium. On concentre à sec, reprend au chlorure de méthylène, lave à l'eau, sèche, filtre et amène à sec.

### b) Désacétylation

On dissout 3 g du produit obtenu dans 30 ml de méthanol et agite à la température ambiante pendant 15 heures et concentre à sec. On obtient 2,7 g de produit désacétylé.

### c) Hydrogénolyse

On dissout le produit obtenu au stade b) dans 100 ml de méthanol. On hydrogénolyse en présence de 600 mg de palladium à 10 % sur charbon actif. On filtre, rince au méthanol et au chlorure de méthylène puis concentre à sec le filtrat. On obtient 2,52 g d'un produit que l'on purifie en éluant avec le mélange éther isopropylique-méthanol-triéthylamine (80-10-10). On obtient 941,8 mg d'un produit que l'on chromatographie à nouveau en éluant avec le mélange éther isopropylique-méthanol-triéthylamine (80-10-10). On obtient ainsi 761 mg de 6-O-méthyl-12,11-(oxycarbonyl) (2-hydrazono)) érythromycine.

| Microanalyse | Calculé | Trouvé |
|---|---|---|
| C % | 59,45 | 58,8 |
| H % | 8,83 | 8,5 |
| N % | 5,33 | 5,2 |

### Spectre de masse

788⁺ = MH⁺
810⁺ = MNa⁺

### STADE B : 11,12-didéoxy 6-O-méthyl 12,11-(oxycarbonyl-(2-(3-(4-quinoléinyl) propyl) hydrazono) érythromycine

On agite 15 heures à la température ambiante un mélange de 230 mg du produit obtenu au stade A ci-dessus, 5 ml de méthanol, 0,3 g de quinoléine 4-propanal, 0,055 ml d'acide acétique. On ajoute 0,065 g de cyanoborohydrure de sodium. On agite à la température ambiante pendant 24 heures. On évapore le méthanol, extrait à l'acétate d'éthyle, lave à l'aide d'une solution de soude puis à l'eau, sèche, filtre et évapore à sec. On obtient 220 mg d'un produit que l'on chromatographie sur silice en éluant avec le mélange acétate d'éthyle-triéthylamine (95-5). On obtient 80 mg du produit recherché.

| Microanalyse | Calculé | Trouvé |
|---|---|---|
| C % | 63,99 | 64,1 |
| H % | 8,42 | 8,3 |
| N % | 5,85 | 5,7 |

### Spectre de masse

158⁺ = OH en 2'
957⁺ = (M+H)⁺
979⁺ = (M⁺Na)⁺

### EXEMPLE 2 : 11,12-didéoxy-3-de((2,6-didéoxy-3-C-méthyl-3-O-méthyl-alpha-L-ribohexopyranosyl)oxy)-3-hydroxy-6-O-méthyl-12,11- (oxycarbonyl((4-(4- (3-pyridinyl) -1H-imidazol-1-yl)-butyl)imino))-érythromycine

En opérant comme à l'exemple 1 à partir de la 11,12-didéoxy-6-O-méthyl-12,11-(oxycarbonyl((4-(4-(3-pyridinyl)-1H-imidazol-1-yl)butyl)imino))-érythromycine, on a obtenu le produit recherché. F = 210°C.

Le produit de départ à savoir la 11,12-didéoxy-6-O-méthyl-12,11- (oxycarbonyl ((4-(4-(3-pyridinyl)-1H-imidazol-1-yl)butyl)imino))-érythromycine a été préparé selon le procédé indiqué ci-dessus pour la préparation de produit de départ de l'exemple 1.

### EXEMPLE DE COMPOSITION PHARMACEUTIQUE

On a préparé des composés renfermant :

| | |
|---|---|
| Produit de l'exemple 1 | 150 mg |
| Excipient q.s.p. | 1 g |

Détail de l'excipient : amidon, talc, stéarate de magnésium

### ETUDE PHARMACOLOGIQUE DES PRODUITS DE L'INVENTION

### Méthode des dilutions en milieu liquide

On a préparé une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination, ce qui permet de déterminer les concentrations minimales inhibitrices (C.M.I.) exprimées en microgrammes/cm³.

Les résultats suivants ont été obtenus :

| Souches bactériennes à GRAM+ | | |
|---|---|---|
| Produits | Ex. 1 | Ex. 2 |
| Staphylococcus aureus 011UC4 | 0,3 | - |
| Staphylococcus aureus 011G025I | | |
| Staphylococcus epidennidis 012G011I | | |
| Strepcococcus pyogenes groupe A 02A1UC1 | 0,04 | 0,15 |
| Streptococcus agalactiae groupe B 02B1HT1 | s 0,02 | 0,04 |
| Streptococcus faecalis groupe D 02D2UC1 | 0,04 | 0,3 |
| Streptococcus faecium groupe D 02D3HT1 | 0,04 | 0,3 |
| Streptococcus sp groupe G 02GOGR5 | 0,04 | 0,15 |
| Streptococcus mitis 02mitCB1 | 0,3 | 0,04 |
| Streptococcus mitis 02mitGR16I | 0,15 | 0,6 |
| Streptococcus agalactiae groupe B 02B1SJ1 | 0,6 | 1,2 |
| Strepcococcus pneumoniae 030SJ5 | 0,6 | 1 |

## Revendications

1. Les composés choisis dans le groupe constitué par les composés de formule (I) : 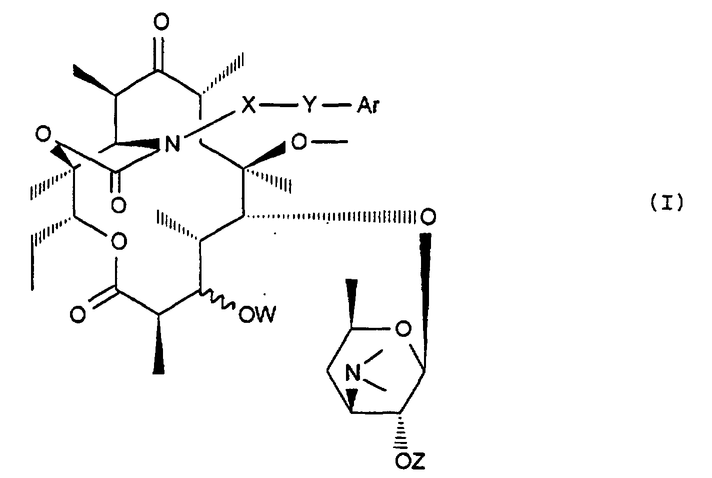 dans lesquels :
X représente un radical CH₂ ou SO₂ ou un atome d'oxygène,
Y représente un radical (CH₂)ₘ-(CH=CH)ₙ(CH₂)ₒ, avec m + n + o ≤ 8, n = 0 ou 1,
Ar représente un radical hétéroaryle éventuellement substitué,
W représente un atome d'hydrogène, ou le reste dune fonction carbamate 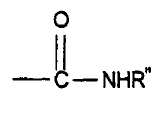 dans lequel R" représente un radical alkyle renfermant jusqu'à 8 atomes de carbone ou un radical aryle, éventuellement substitué,
Z représente un atome d'hydrogène ou le reste d'un acide ainsi que leurs sels d'addition avec les acides, et la 11,12-didéoxy-3-de((2,6-didéoxy-3-C-méthyl-3-O-méthyl-alpha-L-ribohexopyranosyl)oxy)-3-hydroxy-6-O-méthyl-12,11-(oxycarbonyl(2-(3-4-(quinoléinyl)propyl) hydrazono)) érythromycine.

2. Les composés de formule (I) tels que définis à la revendication 1, dans lesquels Z représente un atome d'hydrogène.

3. Les çomposés de formule (I) tels que définis à la revendication 1 ou 2, dans lesquels W représente un atome d'hydrogène.

4. Les composés de formule (I) tels que définis à l'une quelconque des revendications 1 à 3 dans lesquels X représente un radical CH₂.

5. Les composés de formule (I) tels que définis à l'une quelconque des revendications 1 à 4, dans lesquels Y représente un radical (CH₂)₃, (CH₂)₄ ou (CH₂)₅.

6. Les composés de formule (I) tels que définis à l'une quelconque des revendications 1 à 5, dans lesquels Ar représente un radical 4-quinoléinyle éventuellement mono ou poly-substitué sur l'un et/ou l'autre des 2 cycles de la quinoléine.

7. Les composés de formule (I) tels que définis à la revendication 6, dans lesquels Ar représente un radical 4-quinoléinyle non substitué.

8. Les composés de formule (I) tels que définis à la revendication 5, dans lesquels Ar représente un radical : 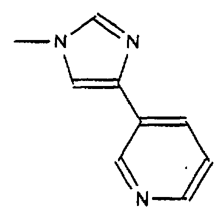 éventuellement substitué.

9. Les composés de formule (I) tels que définis à la revendication 1, dont les noms suivent :
- 11,12-didéoxy-3-de((2,6-didéoxy-3-C-méthyl-3-O-méthyl-alpha-L-ribohexopyranosyl)oxy)-3-hydroxy-6-O-méthyl-12,11-(oxycarbonyl((4-(4-(3-pyridinyl)-1H-imidazol-1-yl)butyl)-imino))-érythromycine,
- 11,12-didéoxy-3-de((2,6-didéoxy-3-C-méthyl-3-O-méthyl-alpha-L-ribohexopyranosyl)oxy)-3-hydroxy-6-O-méthyl-12,11-(oxycarbonyl(2-(3-(4-quinoléinyl)propyl)hydrazono)) érythromycine.

10. A titre de médicaments, les composés de formule (I) tels que définis à l'une quelconque des revendications 1 à 8 ainsi que leurs sels d'addition avec les acides pharmaceutiquement acceptables.

11. A titre de médicaments les composés de formule (I) tels que définis à la revendication 9, ainsi que leurs sels d'addition avec les acides pharmaceutiquement acceptables.

12. Les compositions pharmaceutiques renfermant comme principe actif au moins un médicament selon la revendication 10 ou 11.

13. Procédé de préparation des composés de formule (I) tels que définis à l'une quelconque des revendications 1 à 9, **caractérisé en ce que** l'on soumet un composé de formule (II) : 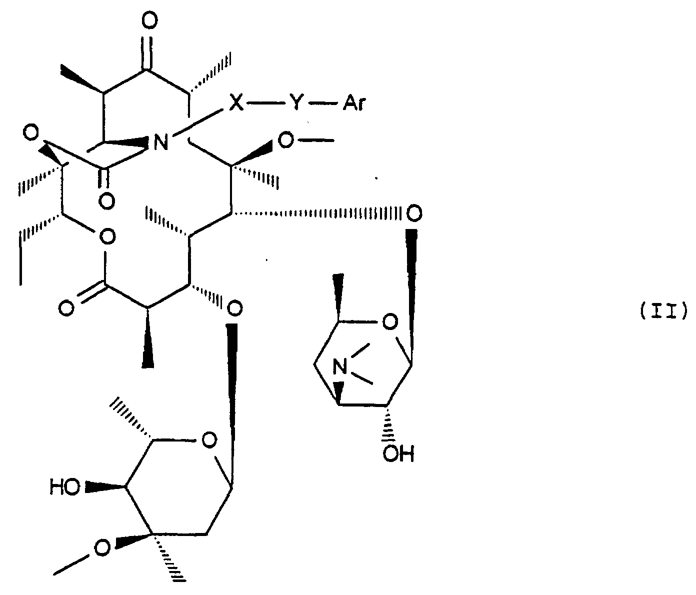 dans lequel X, Y et Ar sont définis comme dans la revendication 1, à l'action d'un agent de clivage du cladinose pour obtenir le composé de formule (I_{A}) correspondant : 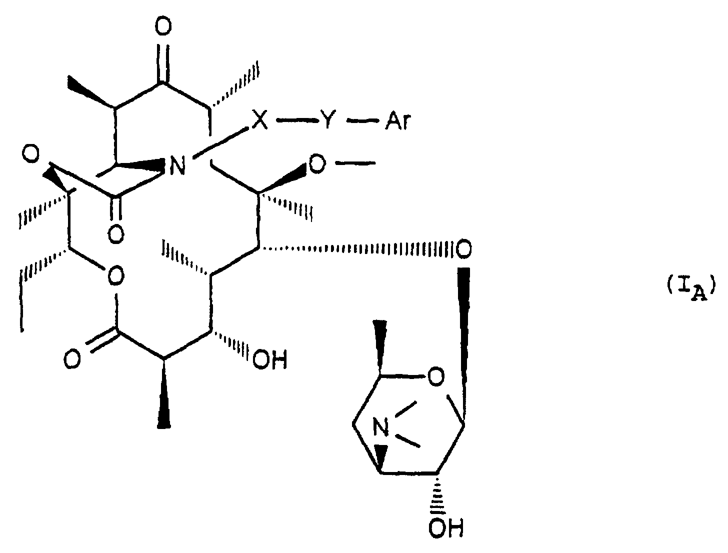 dans lequel X, Y et Ar conservent leur signification précédente que l'on soumet si désiré à l'action d'un agent d'estérification ou à l'action d'un agent susceptible d'introduire le radical carbamate.

## Claims

1. The compounds chosen from the group constituted by the compounds of formula (I): 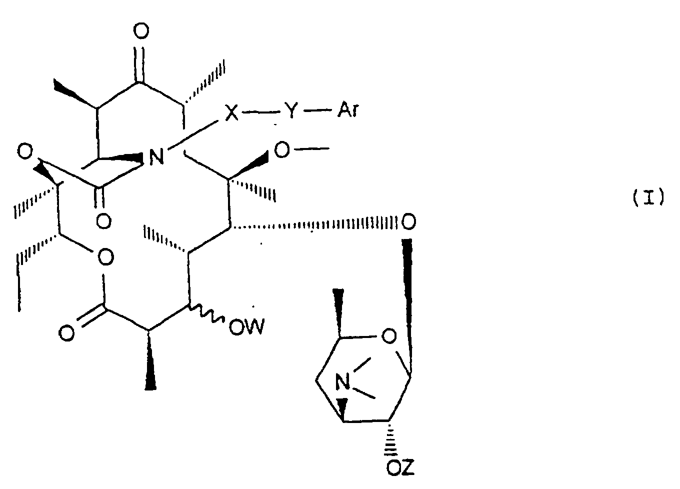 in which:
X represents a CH₂ or SO₂ radical or an oxygen atom,
Y represents a (CH₂)ₘ-(CH=CH)ₙ(CH₂)ₒ, with m +n + o ≤ 8, n = 0 or 1,
Ar represents an optionally substituted heteroaryl radical,
W represents a hydrogen atom, or the remainder of a carbamate function 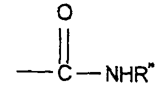 in which R" represents an alkyl radical containing up to 8 carbon atoms or an ary radical, optionally substituted,
Z represents a hydrogen atom or the remainder of an acid as well as their addition salts with acids, and 11, 12-dideoxy-3-de((2,6-dideoxy-3-C-methyl-3-O-methyl-alpha-L-ribohexopyranosyl)oxy)-3-hydroxy-6-O-methyl-12,11-(oxycarbonyl(2-(3-4-(quinolinyl)propyl) hydrazono)) erythromycin.

2. The compounds of formula (I) as defined in claim 1, in which Z represents a hydrogen atom.

3. The compounds of formula (I) as defined in claim 1 or 2, in which W represents a hydrogen atom.

4. The compounds of formula (I) as defined in any one of claims 1 to 3 in which X represents a CH₂ radical.

5. The compounds of formula (I) as defined in any one of claims 1 to 4, in which Y represents a (CH₂)₃, (CH₂)₄ or (CH₂)₅ radical.

6. The compounds of formula (I) as defined in any one of claims 1 to 5, in which Ar represents a 4-quinolinyl radical optionally mono or poly substituted on one or other of the 2 quinoline rings.

7. The compounds of formula (I) as defined in claim 6, in which Ar represents a non-substituted 4-quinolinyl radical.

8. The compounds of formula (I) as defined in claim 5, in which Ar represents an optionally substituted: 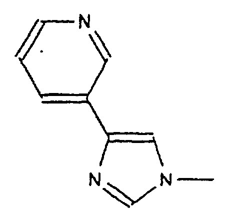 radical.

9. The compounds of formula (I) as defined in claim 1, the names of which follow:
- 11,12-dideoxy-3-de((2,6-dideoxy-3-C-methyl-3-O-methyl-alpha-L-ribohexopyranosyl)oxy)-3-3-hydroxy-6-O-methyl-12,11-(oxycarbonyl((4-(4-(3-pyridinyl)-1H-imidazol-1-yl)butyl)-imino))-erythromycin,
- 11,12-dideoxy-3-de((2,6-dideoxy-3-C-methyl-3-O-methyl-alpha-L-ribohexopyranosyl)oxy)-3-hydroxy-6-O-methyl-12,11-(oxycarbonyl(2-(3-(4-quinolinyl)propyl)hydrazono)) erythromycin.

10. As medicaments, the compounds of formula (I) as defined in any one of claims 1 to 8 as well as their addition salts with pharmaceutically acceptable acids.

11. As medicaments the compounds of formula (I) as defined in claim 9, as well as their addition salts with pharmaceutically acceptable acids.

12. The pharmaceutical compositions containing at least one medicament according to claim 10 or 11 as active ingredient.

13. Process for the preparation of the compounds of formula (I) as defined in any one of claims 1 to 9, **characterised in that** a compound of formula (II): 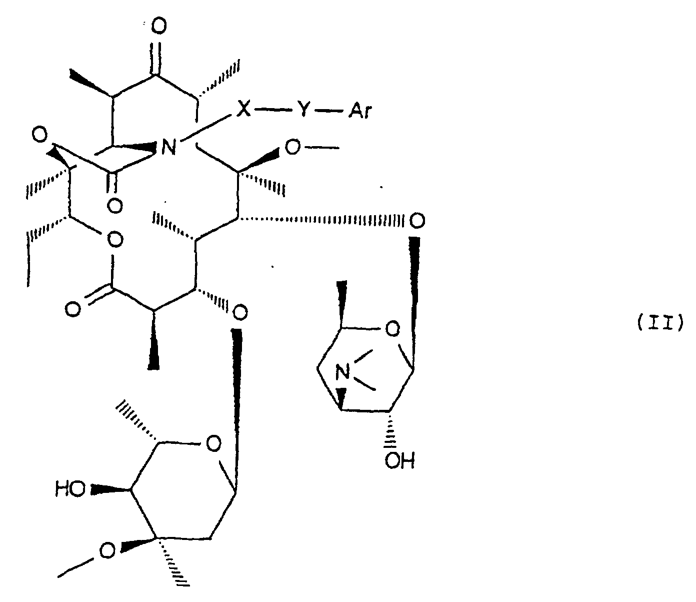 in which X,Y and Ar are as defined in claim 1, is subjected to the action of a cleavage agent of cladinose in order to obtain the corresponding compound of formula (I_{A}) : 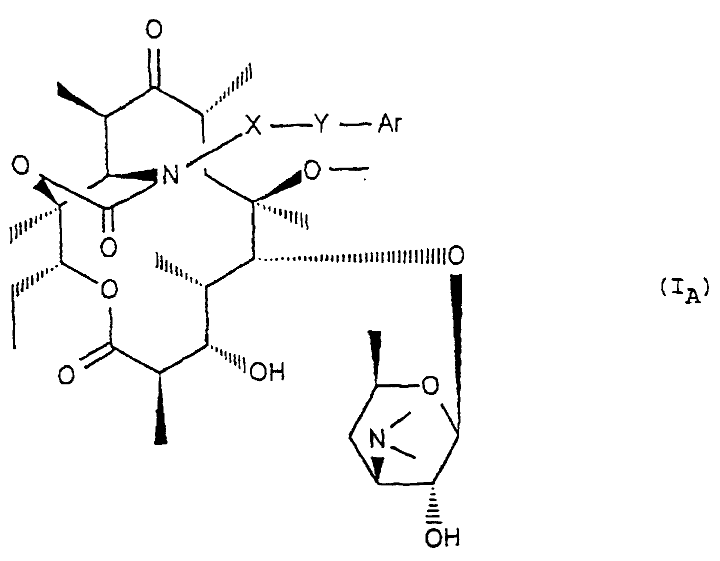 in which X, Y and Ar retain their previous meaning and which are subjected, if necessary, to the action of an esterification agent or to the action of an agent capable of introducing the carbamate radical.

## Patentansprüche

1. Verbindungen, gewählt aus der Gruppe, die aus den Verbindungen der Formel (I) besteht: 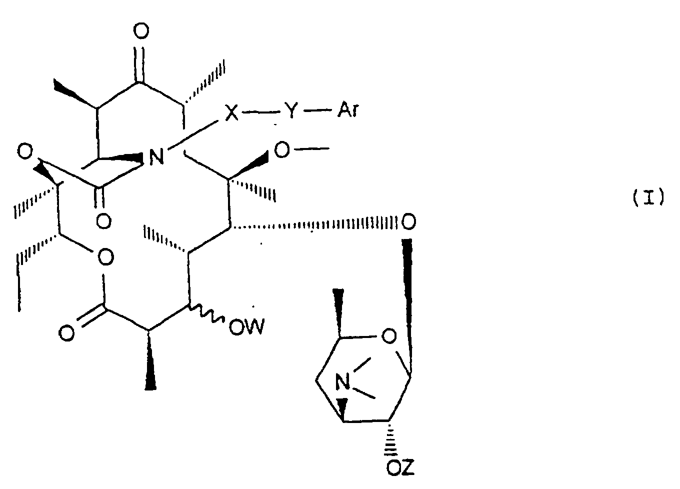 in der
X einen Rest CH₂ oder SO₂ oder ein Sauerstoffatom darstellt,
Y einen Rest (CH₂)ₘ-(CH=CH)ₙ(CH₂)ₒ mit m + n + o ≤ 8, n = 0 oder 1 bedeutet,
Ar ein gegebenenfalls substituierter Rest Heteroaryl ist,
W ein Wasserstoffatom oder den Rest einer Carbamatfunktion 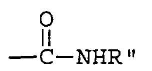 darstellt, worin R" ein Rest Alkyl mit bis zu 8 Kohlenstoffatomen oder ein gegebenenfalls substituierter Rest Aryl ist,
Z ein Wasserstoffatom oder den Rest einer Säure bedeutet, sowie ihre Additionssalze mit Säuren, und
11,12-Dideoxy-3-de-[(2,6-dideoxy-3-C-methyl-3-O-methyl-alpha-Lribohexopyranosyl)-oxy]-3-hydroxy-6-O-methyl-12,11-{oxycarbonyl-{2-[3-4-(chinolinyl)-propyl]-hydrazono}}-erythromycin.

2. Verbindungen der Formel (I) wie in Anspruch 1 definiert, worin Z ein Wasserstoffatom darstellt.

3. Verbindungen der Formel (I) wie in Anspruch 1 oder 2 definiert, worin W ein Wasserstoffatom darstellt.

4. Verbindungen der Formel (I) wie in irgendeinem der Ansprüche 1 bis 3 definiert, worin X einen Rest CH₂ darstellt.

5. Verbindungen der Formel (I) wie in irgendeinem der Ansprüche 1 bis 4 definiert, worin Y einen Rest (CH₂)₃, (CH₂)₄ oder (CH₂)₅ darstellt.

6. Verbindungen der Formel (I) wie in irgendeinem der Ansprüche 1 bis 5 definiert, worin Ar einen Rest 4-Chinolinyl darstellt, gegebenenfalls mono- oder polysubstituiert an dem einen und/oder dem anderen der zwei Ringe des Chinolins.

7. Verbindungen der Formel (I) wie in Anspruch 6 definiert, worin Ar einen nicht substituierten Rest 4-Chinolinyl darstellt.

8. Verbindungen der Formel (I) wie in Anspruch 5 definiert, worin Ar einen gegebenenfalls substituierten Rest 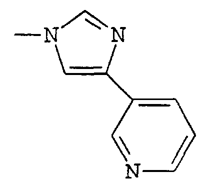 darstellt.

9. Verbindungen der Formel (I) wie in Anspruch 1 definiert, mit den folgenden Namen:
- 11,12-Dideoxy-3-de-[(2,6-dideoxy-3-C-methyl-3-O-methyl-alpha-Lribohexopyranosyl)-oxy]-3-hydroxy-6-O-methyl-12,11-{oxycarbonyl-{[4-[4-(3-pyridinyl)-1H-imidazol-1-yl]-butyl]-imino}}-erythromycin,
- 11,12-Dideoxy-3-de-[(2,6-dideoxy-3-C-methyl-3-O-methyl-alpha-Lribohexopyranosyl)-oxy]-3-hydroxy-6-O-methyl-12,11-{oxycarbonyl-{2-[3-(4-chinolinyl)-propyl]-hydrazono}}-erythromycin.

10. Als Arzneimittel die Verbindungen der Formel (I) wie in irgendeinem der Ansprüche 1 bis 8 definiert sowie ihre Additionssalze mit pharmazeutisch akzeptablen Säuren.

11. Als Arzneimittel die Verbindungen der Formel (I) wie in Anspruch 9 definiert sowie ihre Additionssalze mit pharmazeutisch akzeptablen Säuren.

12. Pharmazeutische Zusammensetzungen, umfassend als Wirkstoff mindestens ein Arzneimittel nach Anspruch 10 oder 11.

13. Verfahren zur Herstellung von Verbindungen der Formel (I) wie in irgendeinem der Ansprüche 1 bis 9 definiert, **dadurch gekennzeichnet, daß** man eine Verbindung der Formel (II) 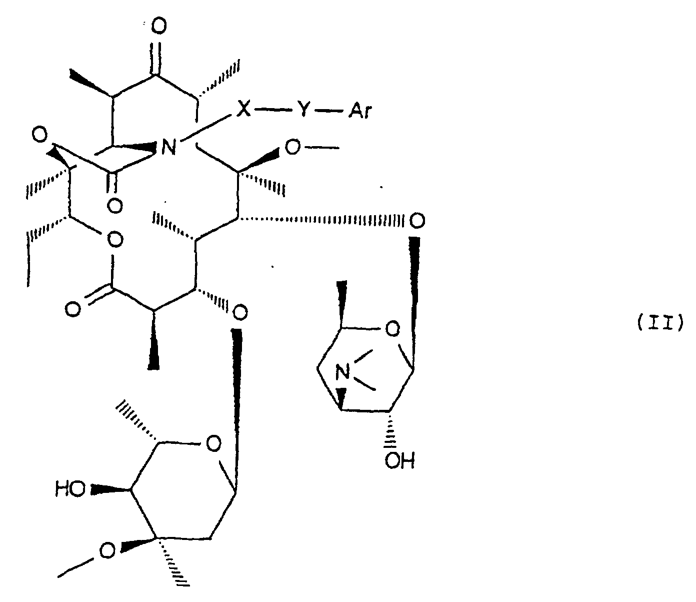 in der X, Y und Ar wie in Anspruch 1 definiert sind, der Einwirkung eines Mittels zur Aufspaltung der Cladinose unterzieht, um die entsprechende Verbindung der Formel (I_{A}) 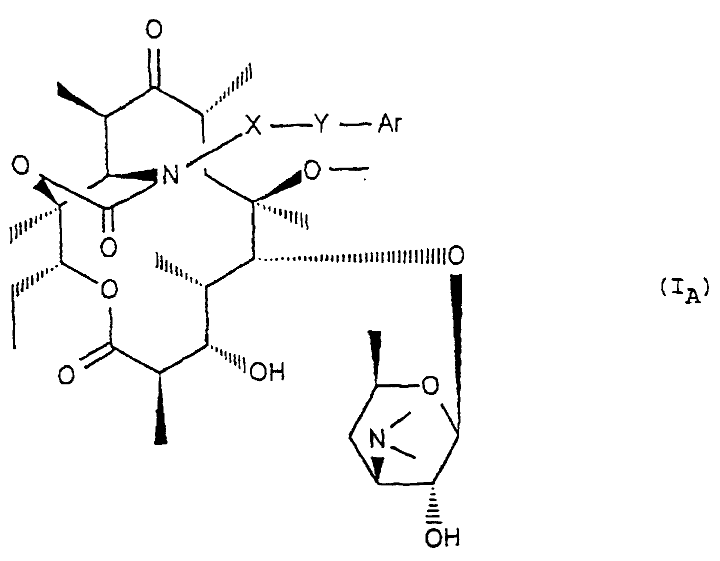 zu erhalten, in der X, Y und Ar ihre vorstehende Bedeutung beibehalten, die man anschließend, wenn erwünscht, der Einwirkung eines Mittels zur Veresterung oder der Einwirkung eines geeigneten Mittels zur Einführung des Carbamat-Restes unterzieht.
